# EUROPÄISCHE PATENTSCHRIFT

(11) **EP 1 169 998 B1**
(45) Veröffentlichungstag und Bekanntmachung des Hinweises auf die Patenterteilung: **29.11.2006**
(21) Anmeldenummer: 01112311.4
(22) Anmeldetag: 19.05.2001
(51) Int. Cl.: A61K 8/03, A61Q 5/00

(54) **Klares, zweiphasiges, schaumbildendes Aerosol-Haarpflegeprodukt**
Clear, two phase foamable aerosol hair care product
Produit aérosol, clair, biphasique et moussant pour le soin des cheveux

(30) Priorität: 08.07.2000 DE 10033414
(43) Veröffentlichungstag der Anmeldung: 09.01.2002
(73) Patentinhaber: Wella Aktiengesellschaft, 64274 Darmstadt (DE)
(72) Erfinder: Keller, Walter, 64372 Ober-Ramstadt (DE); Kischka, Karl-Heinz, 64293 Darmstadt (DE)

(56) Entgegenhaltungen:
- EP-A- 0 418 676
- WO-A-95/22312
- WO-A-99/11222
- WO-A-99/15135
- US-A- 3 433 868
- US-A- 5 059 414

## Beschreibung

Gegenstand der Erfindung ist ein Produkt zur Haarbehandlung bestehend aus einer transparenten Druckverpackung, einer Vorrichtung zum Verschäumen sowie einer aus zwei klaren, scharf voneinander getrennten flüssigen Phasen bestehenden Wirkstoffzusammensetzung. Die Wirkstoffzusammensetzung wird der Druckverpackung als Schaum entnommen, auf dem Haar verteilt und kann sowohl als Leave-in-Produkt angewendet werden und auf dem Haar verbleiben oder sie kann als Rinse-Produkt angewendet und nach einer Einwirkzeit aus dem Haar wieder ausgespült werden.

Haarpflegemittel, die Druckgaspackungen als Schaum entnommen werden, sind bekannt und werden auch als Aerosolschäume, Schaumaerosole oder als Pflegeschäume bezeichnet. Diese Produkte bestehen im wesentlichen aus einer Druckverpackung, einem Schaumkopf, einer flüssigen wässrigen Wirkstoffphase und einer verflüssigten Treibgasphase. In der Regel benötigen diese Produkte eine undurchsichtige Verpackung, beispielsweise aus Metall oder undurchsichtigem Kunststoff, da sich an der Phasengrenze zwischen verflüssigter Treibgasphase und Wirkstoffphase aufgrund von Wechselwirkungen von Treibmittel, Wirk- und Hilfstoffen und Lösungsmitteln eine inhomogene, unkosmetisch und unattraktiv aussehende, trübe "schlunzige", Schlieren bildende Masse bildet und/oder es zu Trübungen von einer oder beiden Phasen aufgrund des Zusammenwirkens der üblicherweise eingesetzten Inhaltsstoffe kommt. Wünschenswert sind aber transparente Verpackungen aufgrund der einfachen optischen Erkennbarkeit des Füllstandes, der einfachen optischen Erkennbarkeit einer ausreichenden Phasendurchmischung nach dem Schütteln vor der Anwendung, der Erkennbarkeit einer gegebenenfalls farblichen Gestaltung der Inhaltsstoffe sowie allgemein wegen einer größeren Attraktivität von klaren, transparenten Produkten.

In der US 5,059,414 werden Produkte zur Haarbehandlung beschrieben, welche in zwei separaten, hochviskosen Phasen vorliegen. Die Stabilität der Phasen wird durch Verdicker gewährleistet. In der US 3,433,868 und in der EP 0 418 676 A1 werden Produkte zur Haarbehandlung beschrieben, welche in einem Druckbehälter als zwei flüssige Phasen vorliegen, wobei eine Phase eine Wasserphase und die andere Phase eine Treibgasphase ist. In der WO 99/11222 wird ein haarpflegendes Zweikomponentenmittel aus zwei bis zur Anwendung getrennt gehaltenen Komponenten beschrieben. In der WO 95/22312 werden Haarbehandlungsmittel beschrieben, welche in einer Ausführungsform als Schaumaerosole vorliegen und flüssige Treibgase enthalten können. In der WO 99/15135 werden Haarstylingmittel beschrieben, welche wasserlösliche, nichtpolymere Mineralsalze und lipophile Stoffe enthalten. Die Mittel können u.a. als Schaum vorliegen.

Es bestand daher die Aufgabe, ein unter Druck stehendes transparentes Haarpflegeprodukt zur Bildung von Schäumen zur Verfügung zu stellen, welches sich durch eine nach dem Schütteln sich reversibel wieder ausbildende scharfe, schlierenfreie und von aussen erkennbare Phasentrennung zwischen verflüssigter Treibgasphase und wasserhaltiger Wirkstoffphase auszeichnet. Die typischen haarpflegenden Eigenschaften der bekannten herkömmlichen Pflegeschäume sollen dabei selbstverständlich nicht oder zumindest nicht wesentlich beeinträchtigt werden.

Es wurde nun gefunden, daß die Aufgabe gelöst wird durch ein Produkt zur Haarbehandlung bestehend aus
(A) einer transparenten, druckfesten Aerosolverpackung,
(B) einer Vorrichtung zum Verschäumen einer in der Aerosolverpackung befindlichen Zusammensetzung,
(C) einer aus zwei flüssigen Phasen (D) und (E) bestehenden Wirkstoffmischung, welche bei Abgabe aus der Verpackung (A) mittels der Vorrichtung (B) einen Schaum bildet, wobei
   (c1) beide flüssigen Phasen klar vorliegen,
   (c2) beide flüssigen Phasen durch eine scharfe Phasengrenze voneinander getrennt vorliegen,
(D) eine der beiden flüssigen Phasen hydrophil ist und Wasser oder ein aus Wasser und wasserlöslichen organischen Lösungsmitteln gebildetes Lösungsmittelsystem enthält,
   (d1) die hydrophile Phase mindestens einen kationischen oder kationaktiven haarpflegenden Stoff enthält,
   (d2) die hydrophile Phase mindestens ein anorganisches Salz oder ein nicht-polymeres organisches Salz enthält, wobei das Kation des Salzes ein Metallion oder das Ammoniumion ist,
(E) die zweite der beiden flüssigen Phasen hydrophob ist und
   (e1) mindestens ein wasserunlösliches und unter den in der Aerosolverpackung (A) herrschenden Druckverhältnissen in verflüssigter Form vorliegendes Treibgas enthält und
   (e2) die hydrophobe Phase mindestens eine wasserunlösliche, in dem verflüssigten Treibgas (e1) lösliche hydrophobe Substanz enthält.

Das Salz (d2) und die hydrophobe Substanz (e2) bewirken zwar jede für sich alleine bereits eine gewisse Verbesserung der Schärfe der Phasentrennung. Die erwünschte, besonders scharfe Phasentrennung wird aber erst durch eine synergistische Wirkung bei gleichzeitigem Zusatz der Salze (d2) zu der hydrophilen Phase und der hydrophoben Substanz (e2) zu der hydrophoben Phase erreicht (siehe Vergleichsversuche in Beispiel 6).

Der kationische oder kationaktive haarpflegende Stoff (d1) ist in der Zusammensetzung des erfindungsgemäßen Produktes vorzugsweise in einer Menge von 0,05 bis 10, besonders bevorzugt von 0,1 bis 5 Gew.% enthalten. Das Salz (d2) ist vorzugsweise in einer Menge von 0,05 bis 2, besonders bevorzugt von 0,1 bis 1,0 Gew.% enthalten.

Die öl- oder fettartige Substanz (e2) ist vorzugsweise in einer Menge von 0,5 bis 10, besonders bevorzugt von 1 bis 5 Gew.% enthalten. Die Mengenangaben beziehen sich jeweils auf die Gesamzusammensetzung.

Das erfindungsgemäße Produkt ist in einer geeigneten, druckdichten Aerosolverpackung verpackt und weist als zusätzliche Komponente eine Vorrichtung zum Verschäumen der in der Verpackung enthaltenen Zusammensetzung auf, welche das Verschäumen der Zusammensetzung unter Verwendung des Treibmittels ermöglicht. Als geeignete Schäumvorrichtung kann beispielsweise ein handelsüblicher Aerosolschaumkopf verwendet werden. Die Verpackung ist aus einem transparenten Material, durch welches die Konsistenz, die Füllstandshöhe, die Farbe der Zusammensetzung und der Durchmischungsgrad bzw. die Trennung der beiden flüssigen Phasen erkennbar ist. Als Materialien für die Verpackung kommen beispielsweise Glas und transparente, druckfeste Kunststoffe infrage, wobei Kunststoffe aus Kosten- und Gewichtsgründen bevorzugt sind. Besonders bevorzugt ist Polyethylenterephtalat.

Die beiden flüssigen Phasen sind klar im Sinne der Erfindung, wenn mit bloßem Auge keine Trübungen oder Schlieren zu erkennen sind. Die Phasen sind im Sinne der Erfindung dann scharf voneinander getrennt, wenn im ungeschüttelten Ruhezustand mit bloßem Auge keine Grenzschicht sondern lediglich eine Trennlinie zwischen den Phasen erkennbar ist bzw. die Trennlinie zwischen den Phasen kleiner 1 mm, vorzugsweise kleiner 0,1 mm ist.

### Lösungsmittel der hydrophilen Phase

Die hydrophile Phase enthält Wasser entweder als einziges Lösungsmittel oder im Gemisch mit Alkoholen. Als Alkohole können insbesondere die für kosmetische Zwecke üblicherweise verwendeten niederen einwertigen Alkohole mit 1 bis 4 Kohlenstoffatomen wie zum Beispiel Ethanol und Isopropanol enthalten sein oder mehrwertige Alkohole mit 2 bis 6 Kohlenstoffatomen wie Ethylenglykol, Glycerin, Propylenglykol, Butylenglykol oder Pentandiol. Der Wassergehalt beträgt vorzugsweise von 55 bis 98, besonders bevorzugt von 60 bis 95 Gew.% und der Alkoholgehalt beträgt vorzugsweise von 0 bis 40, besonders bevorzugt von 1 bis 20 Gew.%, jeweils bezogen auf die Gesamtzusammensetzung.

Die hydrophile Phase liegt vorzugsweise in einem pH-Bereich von 2 bis 7, besonders bevorzugt von 4 bis 6,5 vor. Dabei kann ein saurer Bereich eingestellt werden mit einer kosmetisch verträglichen organischen oder anorganischen Säure wie beispielsweise Ameisensäure, Weinsäure, Äpfelsäure, Maleinsäure, Fumarsäure, Pyrrolidoncarbonsäure, Zitronensäure, Milchsäure, Schwefelsäure, Essigsäure, Salzsäure, Phosphorsäure u.a..

### Kationischer, haarpflegender Stoff

Der kationische oder kationaktive Stoff (d1) ist eine Substanz, die auf Grund von kationischen oder kationisierbaren Gruppen, insbesondere protonierten Amingruppen oder quaternären Ammoniumgruppen eine Substantivität zu menschlichem Haar aufweist. Der kationische oder kationaktive haarpflegende Stoff (d1) ist vorzugsweise ausgewählt aus kationischen Polymeren, kationischen Tensiden, kationischen Silikonverbindungen, kationisch derivatisierten Proteinen, kationisch derivatisierten Proteinhydrolysaten und Betain mit jeweils mindestens einer kationischen oder kationaktiven Gruppe.

Besonders gute haarpflegende Wirkungen werden erzielt, wenn mindestens ein kationisches Polymer mit mindestens einem kationischen Tensid kombiniert wird. Die besten Wirkungen wurden erhalten, wenn zusätzlich noch mindestens eine kationische Silikonverbindung, insbesondere ein endständig diquaternäres Polydimethylsiloxan enthalten ist.

Geeignete kationische Tenside sind Tenside, welche eine quaternäre Ammoniumgruppe enthalten. Geeignete kationische Tenside sind insbesondere solche der allgemeinen Formel (I)

N⁽⁺⁾R¹R²R³R⁴ X⁽⁻⁾ (I)

wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten, wobei mindestens einer der Reste R1 bis R4 mindestens 8 C-Atome aufweist und wobei X⁻ ein Anion darstellt, beispielsweise ein Halogen, Acetat, Phosphat, Nitrat oder Alkylsulfat, vorzugsweise ein Chlorid. Die aliphatischen Gruppen können zusätzlich zu den Kohlenstoffatomen und den Wasserstoffatomen auch Querverbindungen oder andere Gruppen wie beispielsweise weitere Aminogruppen enthalten.

Beispiele für geeignete kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, Alkylamidoethyltrimethylammoniumethersulfate sowie Verbindungen mit kationischem Charakter wie Aminoxide, beispielsweise Alkylmethylaminoxide oder Alkylaminoethyldimethylaminoxide. Besonders bevorzugt ist Cetyltrimethylammoniumchlorid.

Bei den geeigneten kationischen oder kationaktiven Polymeren handelt es sich um haarpflegende, bzw. haarkonditionierende Polymere. Geeignete kationische Polymere enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium-11) sowie quaternäre Silikonpolymere bzw. -oligomere wie beispielsweise Silikonpolymere mit quaternären Endgruppen (Quaternium-80).

Von den kationischen Polymeren, die in dem erfindungsgemäßen Mittel enhalten sein können, ist zum Beispiel Vinylpyrrolidon/Dimethylaminoethylmethacrylatmethosulfat Copolymer, das unter den Handelsbezeichnungen Gafquat^{®} 755 N und Gafquat^{®} 734 vertrieben wird und von denen das Gafquat^{®} 755 N besonders bevorzugt ist, geeignet. Weitere kationische Polymere sind beispielsweise das unter dem Handelsnamen LUVIQUAT^{®} HM 550 vertriebene Copolymer aus Polyvinylpyrrolidon und Imidazoliminmethochlorid, das unter dem Handelsnamen Merquat^{®} Plus 3300 vertriebene Terpolymer aus Dimethyldiallylammoniumchlorid, Natriumacrylat und Acrylamid, das unter dem Handelsnamen Gaffix^{®} VC 713 vertriebene Terpolymer aus Vinylpyrrolidon, Dimethylaminoethylmethacrylat und Vinylcaprolactam und das unter dem Handelsnamen Gafquat^{®} HS 100 vertriebene Vinylpyrrolidon/Methacrylamidopropyltrimethylammoniumchlorid Copolymer.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel (II)

G-O-B-N+R⁵R⁶R⁷ X⁻ (II)

G ist ein Anhydroglucoserest, beispielsweise Stärke-oder Celluloseanhydroglucose;
B ist eine divalente Verbindungsgruppe, beispielsweise Alkylen, Oxyalkylen, Polyoxyalkylen oder Hydroxyalkylen;
R⁵, R⁶ und R⁷ sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R⁵, R⁶ und R⁷ vorzugsweise maximal 20 ist;
X ist ein übliches Gegenanion, hat die gleiche Bedeutung wie bei Formel (I) und ist vorzugsweise Chlorid. Eine kationische Cellulose wird unter der Bezeichnung Polymer JR von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Ein geeignetes kationisches Guarderivat wird unter der Handelsbezeichnung Jaguar^{®} R vertrieben und hat die INCI-Bezeichnung Guar Hydroxypropyltrimonium Chloride.

Besonders bevorzugte kationaktive Stoffe sind Chitosan, Chitosansalze und Chitosan-Derivate. Bei den erfindungsgemäß einzusetzenden Chitosanen handelt es sich um vollständig oder partiell deacetylierte Chitine. Zur Herstellung von Chitosan geht man vorzugsweise von dem in den Schalenresten von Krustentieren enthaltenem Chitin aus, welches als billiger und natürlicher Rohstoff in großen Mengen zur Verfügung steht. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%.

Ein geeignetes Chitosan wird beispielsweise von der Firma Kyowa Oil&Fat, Japan, unter dem Handelsnamen Flonac^{®} vertrieben. Es hat ein Molekulargewicht von 300.000 bis 700.000 g/mol und ist zu 70 bis 80% entacetyliert. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosanderivate kommen quaternisierte, alkylierte oder hydroxyalkylierte Derivate, beispielsweise Hydroxyethyl-, Hydroxypropyl- oder Hydroxybutylchitosan in Betracht.

Die Chitosane oder Chitosanderivate liegen vorzugsweise in neutralisierter oder partiell neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Äpfelsäure, Milchsäure, Pyrrolidoncarbonsäure, Salzsäure u.a., von denen die Pyrrolidoncarbonsäure und die Milchsäure besonders bevorzugt sind.

Bevorzugt sind solche Polymere, die eine ausreichende Löslichkeit in Wasser oder in Wasser/Alkohol-Gemischen besitzen, um in der erfindungsgemäßen hydrophilen Phase in vollständig gelöster Form vorzuliegen. Die kationische Ladungsdichte beträgt vorzugsweise 1 bis 7 meq/g.

Geeignete kationaktive Silikonvberbindungen weisen vorzugsweise entweder mindestens eine Aminogruppe oder mindestens eine Ammoniumgruppe auf. Geeignete Silikonpolymere mit Aminogruppen sind unter der INCI-Bezeichnung Amodimethicone bekannt. Hierbei handelt es sich um Polydimethylsiloxane mit Aminoalkylgruppen. Die Aminoalkylgruppen können seiten- oder endständig sein. Geeignete Aminosilikone sind solche der allgemeinen Formel (III)

R⁸R⁹R¹⁰Si-(OSiR¹¹R¹²)ₓ-(OSiR¹³Q)_{y}-OSiR¹⁴R¹⁵R¹⁶ (III)

R⁸, R⁹, R¹⁴ und R¹⁵ sind unabhängig voneinander gleich oder verschieden und bedeuten C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl;
R¹⁰ und R¹⁶ sind unabhängig voneinander gleich oder verschieden und bedeuten -(CH₂)ₐ-NH₂ mit a gleich 1 bis 6, C1- bis C10-Alkyl, Phenyl, Hydroxy, Wasserstoff, C1-bis C10-Alkoxy oder Acetoxy, vorzugsweise C1-C4-Alkyl, besonders bevorzugt Methyl;
R¹¹, R¹² und R¹³ sind unabhängig voneinander gleich oder verschieden und bedeuten Wasserstoff, C1- bis C20-Kohlenwasserstoff, welcher O- und N-Atome enthalten kann, vorzugsweise C1- bis C10-Alkyl oder Phenyl, besonders bevorzugt C1- bis C4-Alkyl, insbesondere Methyl;
Q bedeutet -A-NR¹⁷R¹⁸, oder -A-N⁺R¹⁷R¹⁸R¹⁹ wobei A für eine divalente C1- bis C20-Alkylenverbindungsgruppe steht, welche auch O- und N-Atome sowie OH-Gruppen enthalten kann, und R¹⁷, R¹⁸ und R¹⁹ unabhängig voneinander gleich oder verschieden sind und Wasserstoff, C1- bis C22-Kohlenwasserstoff, vorzugsweise C1- bis C-4-Alkyl oder Phenyl bedeuten. Bevorzugte Reste für Q sind
   - (CH₂)₃-NH₂, - (CH₂)₃NHCH₂CH₂NH₂, -(CH₂)₃OCH₂CHOHCH₂NH₂ und
   - (CH₂)₃N(CH₂CH₂OH)₂, - (CH₂)₃-NH₃⁺ und
   - (CH₂)₃OCH₂CHOHCH₂N⁺(CH₃)₂R²⁰, wobei R²⁰ ein C1- bis C22-Alkylrest ist, der auch OH-Gruppen aufweisen kann.
X bedeutet eine Zahl zwischen 1 und 10.000, vorzugsweise zwischen 1 und 1.000;
Y bedeutet eine Zahl zwischen 1 und 500, vorzugsweise zwischen 1 und 50.

Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 0,1 bis 0,5.

Besonders bevorzugt sind Silikonpolymere mit zwei endständigen quaternären Ammoniumgruppen. Diese Verbindungen sind unter der INCI-Bezeichnung Quaternium-80 bekannt. Hierbei handelt es sich um Polydimethylsiloxane mit zwei endständigen Alkylammoniumgruppen. Geeignete quaternäre Aminosilikone sind solche der allgemeinen Formel (IV)

R²¹R²²R²³N⁺-A-SiR⁸R⁹-(OSiR¹¹R¹²)ₙ-OSiR⁸R⁹-A-N⁺R²¹R²²R²³ 2X⁻ (IV)

A hat die gleiche Bedeutung wie oben bei Formel (III) angegeben und ist vorzugsweise
- (CH₂) ₃OCH₂CHOHCH₂N⁺(CH₃) ₂R²⁰, wobei R²⁰ ein C1- bis C22-Alkylrest ist, der auch OH-Gruppen aufweisen kann; R⁸, R⁹, R¹¹ und R¹² haben die gleiche Bedeutung wie oben bei Formel (III) angegeben und sind vorzugsweise Methyl;
R²¹, R²², und R²³ bedeuten unabhängig voneinander C1-bis C22-Alkylreste, welche Hydroxygruppen enthalten können und wobei vorzugsweise mindestens einer der Reste mindestens 10 C-Atome aufweist und die übrigen Reste 1 bis 4 C-Atome aufweisen; n ist eine Zahl von 0 bis 200, vorzugsweise von 10 bis 100.

Derartige diquaternäre Polydimethylsiloxane werden von der Firma GOLDSCHMIDT/Deutschland unter den Handelsnamen Abil^{®} Quat 3270, 3272 und 3274 vertrieben.

Weitere geeignete kationaktive, haarpflegende Verbindungen sind kationisch modifizierte Proteinderivate oder kationisch modifizierte Proteinhydrolysate und sind beispielsweise bekannt unter den INCI-Bezeichnungen Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein oder Hydroxypropyltrimonium Hydrolyzed Wheat, Hydroxypropyltrimonium Hydrolyzed Casein, Hydroxypropyltrimonium Hydrolyzed Collagen, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, Hydroxypropyltrimonium Hydrolyzed Vegetable Protein.

Geeignete kationisch derivatisierte Proteinhydrolysate sind Substanzmischungen, die beispielsweise durch Umsetzung von alkalisch, sauer oder enzymatisch hydrolysierten Proteinen mit Glycidyltrialkylammoniumsalzen oder 3-Halo-2-hydroxypropyltrialkylammoniumsalzen erhalten werden können. Proteine, die als Ausgangsstoffe für die Proteinhydrolysate dienen, können sowohl pflanzlicher als auch tierischer Herkunft sein. Übliche Ausgangsstoffe sind beispielsweise Keratin, Collagen, Elastin, Sojaprotein, Reisprotein, Milchprotein, Weizenprotein, Seidenprotein oder Mandelprotein. Durch die Hydrolyse entstehen Stoffmischungen mit Molmassen im Bereich von ca. 100 bis ca. 50.000. Übliche mittlere Molmassen liegen im Bereich von etwa 500 bis etwa 1000. Vorteilhafterweise enthalten die kationisch derivatisierten Proteinhydrolysate eine oder zwei lange C8- bis C22-Alkylketten und entsprechend zwei oder eine kurze C1- bis C4-Alkylketten. Verbindungen, die eine lange Alkylkette enthalten, sind bevorzugt.

### Salze

Die erfindungsgemäß einzusetzenden Salze sind in Wasser oder in dem wässrig-alkoholischen Lösungsmittelsystem der hydrophilen Phase löslich. Bei den Kationen der erfindungsgemäß einzusetzenden Salze handelt es sich um Metallkationen oder um das Ammoniumkation NH4⁺. Geeignet sind insbesondere ein- zwei- oder dreiwertige Metallkationen. Bevorzugt sind die Kationen der Alkali- und Erdalkalimetalle, insbesondere Natrium, Kalium, Magnesium und Calcium sowie von Aluminium. Als anorganische Anionen kommen insbesondere Chloride, Bromide, Sulfate, Phosphate, Nitrate, Carbonate und Hydrogencarbonate in Frage, von denen die Chloride, Sulfate, Phosphate und Carbonate besonders bevorzugt sind. Als organische Anionen kommen deprotonierte monomere organische Säuren in Frage, insbesondere deprotonierte Carbonsäuren mit 1 bis 10 C-Atomen sofern sie wasserlöslich sind. Beispiele für organische Anionen sind Formiate, Acetate, Propionate, Lactate, Pantothenate, Tartrate, Malate, Pyrrolidoncarboxylate oder Citrate. Besonders bevorzugte Salze sind Natriumchlorid, Natriumsulfat, Magnesiumsulfat, beispielsweise in Form des Heptahydrats, Ammoniumchlorid, Calciumchlorid, Zinksulfat, Dinatriumhydrogenphosphat, Kaliumaluminiumsulfat, Calciumpanthotenat, Natriumlactat und Calciumacetat, wobei auch Salzgemische mit unterschiedlichen Kationen und Anionen möglich sind.

### Treibgase

Bei den erfindungsgemäß einzusetzenden Treibgasen handelt es sich um hydrophobe Stoffe, welche unter Normalbedingungen, d.h. bei Normaldruck (1013 mbar) und Raumtemperatur (20°C) gasförmig sind, bei Abfüllung unter Druck in verflüssigter Form vorliegen und zumindest teilweise in der hydrophilen Phase unlöslich sind und deshalb eine zweite, hydrophobe flüssige Phase bilden. Geeignet sind insbesondere C3- bis C5-Kohlenwasserstoffe wie Propan, Isobutan, n-Butan, Isopentan oder n-Pentan oder deren Gemische sowie Fluorkohlenwasserstoffe wie F 152 (1,1-Difluorethan) oder F 134 (Tetrafluorethan). Es sind auch Gemische mit hydrophilen, teilweise wasserlöslichen Treibgasen wie z.B. Dimethylether möglich, sofern diese nicht als einziges Treibmittel eingesetzt werden, da sich ansonsten nicht die zweite Phase ausbilden würde. Reiner Dimethylether hat außerdem den Nachteil, dass er mit den besonders bevorzugten Verpackungsmaterialien aus transparentem Kunststoff, insbesondere mit Polyethylenterephtalat nicht verträglich ist. Die Behälterwand kann durch Dimethylether aufgeweicht und instabil werden, Stoffe können aus der Behälterwand herausgelöst werden oder die Diffusion von Inhaltstoffen der Wirkstofflösung in oder durch die Behälterwand kann gefördert werden. Besonders bevorzugt sind Gemische aus Propan und Butan sowie Gemische aus Propan, Butan und Dimethylether.

Die Einsatzmenge der Treibgase wird vorzugsweise so gewählt, dass nach Abfüllung der Druck in der Aerosolverpackung maximal 2,7 bar bei Raumtemperatur (20°C) beträgt. Typische Mengen sind 2 bis 30 Gew.%, vorzugsweise 5 bis 20 Gew.%, bezogen auf die Gesamtzusammensetzung.

### Hydrophobe Substanzen

Ein weiterer wesentlicher Bestandteil der hydrophoben Phase ist mindestens eine wasserunlösliche, in dem verflüssigten Treibgas (e1) lösliche hydrophobe Substanz (e2). Diese hydrophobe Substanz (e2) ist vorzugsweise öl- oder fettartig und ausgewählt aus pflanzlichen, tierischen, synthetischen, oder mineralischen Ölen, flüssigen Kohlenwasserstoffen, insbesondere flüssigen, aliphatischen, gesättigten Kohlenwasserstoffen, Fettalkoholen, Fettalkoholestern, Fettsäuren, Fettsäureestern, insbesondere Ester von Fettsäuren und Monoalkoholen sowie Fettsäureglyceridester, Fettalkoholethern und flüssigen Silikonverbindungen, insbesondere Silikonölen.

Als Mineralöle können eingesetzt werden: Paraffinum liquidum, Paraffinum perliquidum, Paraffinum subliquidum, die überwiegend ein Gemisch verzweigter und cyclischer Alkane darstellen. Die aliphatischen, gesättigten Kohlenwasserstoffe können verzweigt oder unverzweigt sein. Geeignet sind insbesondere Paraffine und Isoparaffine wie Isododecan oder cyclische Kohlenwasserstoffe wie Dioctylcyclohexan etc.. Beispiele für geeignete pflanzliche Öle sind Jojobaöl, Olivenöl, Sonnenblumenöl, Avocadoöl etc.. Beispiele für tierische Öle sind Klauenöl, Lebertran, Nerzöl, Talgöl, Fischöl etc.. Geeignete Fettalkohole und Fettsäuren weisen vorzugsweise 8 bis 22 C-Atome auf und können gesättigt oder ungesättigt, linear oder verzweigt sein, beispielsweise Caprylsäure, Ölsäure, Isostearinsäure etc. bzw. die entsprechenden Fettalkohole. Bei den Fettsäureestern kann es sich um Ester von Fettsäuren mit kurzkettigen Alkoholen (C1- bis C5-Alkoholen) handeln, z.B. Isopropylmyristat, Isopropylpalmitat oder Isobutylpalmitat. Geeignete Fettsäureester sind auch die Mono-, Di- oder Triglyceride. Geeignet sind auch Ester aus Fettsäuren und Fettalkohlen, z.B. Cetyl- oder Stearylisononanoat, Myristylmyristat, etc.. Geeignete Fettalkoholester sind Ester von C8- bis C22-Fettalkoholen mit nicht zu den Fettsäuren zählenden organischen Säuren, z.B. Cetyllactat, Myristyladipat etc.. Geeignete Fettalkoholether sind aufgebaut aus zwei Fettalkohlen, die über eine Ethergruppe verknüpft sind, z.B. Dicaprylether. Als Silikonverbindungen können eingesetzt werden: lineare Polydimethylsiloxane (Dimethicone, Hexamethylsiloxan, etc.), cyclische Dimethylsiloxane (Cyclomethicone), Silikonwachse wie z.B. Cetyldimethicone etc.. Die hydrophoben Substanzen können einzeln oder im Gemisch eingesetzt werden.

Eine besonders geringe Belastung des behandelten Haares wird erreicht, wenn die hydrophobe Phase mindestens eine hydrophobe, flüssige, leicht flüchtige Substanz enthält, die vorzugsweise ausgewählt ist aus flüchtigen Silikonen und flüchtigen Kohlenwasserstoffen. Für im Haar verbleibende Pflegemittel empfiehlt sich der Einsatz von bei Raumtemperatur (20°C) bzw, unter Wärmezufuhr (Fönen von feuchten Haaren) flüchtigen, im verflüssigten Treibgas gelösten Stoffen aus den vorher genannten Substanzklassen, um unerwünschte Belastungserscheinungen des Haares zu verhindern. In einer besonders bevorzugten Ausführungsform ist daher in der hydrophoben Phase mindestens ein leicht flüchtiger Kohlenwasserstoff und/oder mindestens eine leicht flüchtige Silikonverbindung enthalten. Die Zusammensetzung ist dann vorzugsweise frei von schwerflüchtigen hydrophoben Substanzen oder enthält diese nur in einer Menge von 3 oder weniger Gew.%. Leicht flüchtig im Sinne dieser Erfindung sind Stoffe, die bei 100°C innerhalb von 20 Minuten sich restlos von einer in dem Stoff getränkten Haarsträhne verflüchtigen. Bevorzugt sind Stoffe, die bei Normaldruck einen Siedepunkt von unter 250°C aufweisen. Schwer flüchtig im Sinne der Erfindung sind solche Stoffe, die nicht leicht flüchtig gemäß der obigen Definition sind. Geeignete leicht flüchtige Substanzen sind leicht flüchtige Paraffine, leicht flüchtige Isoparrafine oder leicht flüchtige Silikonöle. Bevorzugt sind verzweigte oder unverzweigte Kohlenwasserstoffe mit 6 bis 14 C-Atomen, vorzugsweise mit 8 bis 12 C-Atomen wie z.B. Dekan, Dodekan, Isododekan oder deren weiteren Isomere sowie lineare oder cyclische Dimethylpolysiloxane wie z.B. Dimethicone oder Cyclomethicone.

In der hydrophoben Phase können vorteilhafterweise auch haarpflegende nicht-flüssige Stoffe in gelöster Form vorliegen, beispielsweise Wachse, z.B. Frucht- oder Pflanzenwachse wie Apfelwachs, Ilexharz oder auch Lanolin, Paraffinwachs, Vaseline oder höherviskose Silikonverbindungen und Silikonharze.

### Anfärbung der Phasen

Vorteilhafterweise können die beiden flüssigen Phasen mit Anfärbefarbstoffen verschiedenartig angefärbt sein. Die sich beim Schütteln vor der Anwendung einstellende Mischfarbe ist ein optischer Indikator für eine für die Anwendung ausreichende Vermischung. Hierbei werden in der hydrophoben Phase wasserunlösliche, öllösliche Anfärbefarbstoffe und in der hydrophilen Phase ölunlösliche, wasserlösliche bzw. in Wasser/Alkohol-Gemischen lösliche Anfärbefarbstoffe eingesetzt.

### Nichtionische Polymere

In einer weiteren bevorzugten Ausführungsform enthält das erfindungsgemäße Mittel zur Verbesserung der haarpflegenden Eigenschaften mindestens ein filmbildendes, nicht-ionisches Polymer. Die Einsatzmenge beträgt vorzugsweise 0,05 bis 15 Gew.%, besonders bevorzugt von 0,5 bis 5 Gew.%. Bevorzugt sind insbesondere solche Polymere, die eine ausreichende Löslichkeit in Wasser oder Wasser/Alkohol-Gemischen besitzen, um in der hydrophilen Phase in vollständig gelöster Form vorzuliegen. Unter filmbildenden Polymeren werden erfindungsgemäß solche Polymere verstanden, die bei Anwendung in 0,01 bis 5%-iger wässriger, alkoholischer oder wässrig-alkoholischer Lösung in der Lage sind, auf dem Haar einen Polymerfilm abzuscheiden.

Geeignete synthetische, nichtionische filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignet sind z.B. Homopolymere des Vinylcaprolactams, des Vinylpyrrolidons oder des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, Polyvinylalkohole sowie Polyethylenglykol/Polypropylenglykol-Copolymere. Geeignete natürliche filmbildende Polymere sind z.B. Cellulosederivate, z.B. Hydroxyalkylcellulosen wie Hydroxypropylcellulose mit einem Molekulargewicht von beispielsweise 30.000 bis 50.000 g/mol.Besonders bevorzugt sind Polyvinylpyrrolidon und Polyvinylpyrrolidon/Vinylacetat Copolymere.

### Weitere Zusatzstoffe

Das erfindungsgemäße Mittel kann darüber hinaus die für Haarbehandlungsmittel üblichen Zusatzbestandteile enthalten, zum Beispiel Konservierungsmittel, Emulgatoren, Lösungsvermittler, Parfümöle, Duftstoffe, Verdickungsmittel, pH-Puffersubstanzen, Pflegestoffe wie z.B. Pflanzen- und Kräuterextrakte, Protein- und Seidenhydrolysate, Lichtschutzmittel, Antioxidantien, Radikalfänger, Antischuppenwirkstoffe, Glanzgeber, Vitamine, Weichmacher, Kämmbarkeitsverbesserer etc..

Eine besonders bevorzugte Ausführungsform weist eine Wirkstoffzusammensetzung mit der folgenden Zusammensetzung auf:
(a) 0,05 bis 5,0 Gew.% mindestens eines wasserlöslichen, nichtionischen, filmbildenden Polymers
(b) 0,05 bis 5,0 Gew.% mindestens eines wasserlöslichen kationischen Polymers und/oder mindestens eines diquaternären Polydimethylsiloxans,
(c) 0,05 bis 2,5 Gew.% mindestens eines wasserlöslichen kationischen Tensids
(d) 0,05 bis 2,0 Gew.% mindestens eines wasserlöslichen anorganischen Salzes und/oder eines nicht-polymeren organischen Salzes, wobei das Kation des Salzes ein Metallion oder das Ammoniumion ist
(e) 63,0 bis 97,25 Gew. Wasser oder eines Wasser/ Alkohol-Gemisches,
(f) 0,5 bis 5,0 Gew.% mindestens eines unter Normalbedingungen flüssigen, flüchtigen Kohlenwasserstoffs und/oder eines flüchtigen Silikons und
(g) 2,0 bis 20,0 Gew.% eines Treibmittels, ausgewählt aus Propan, Butanen, deren Gemischen und deren Gemischen mit Dimethylether,
wobei sich die Mengenangaben auf die Gesamtzusammensetzung beziehen.

Das erfindungsmäße Produkt wird hergestellt, indem zunächst die in der hydrophilen Phase löslichen Bestandteile in dem hydrophilen Lösungsmittel gelöst werden. Vor dem Abfüllen mit Treibgas werden die in der hydrophoben Phase löslichen Bestandteile zudosiert. Zum Schluß wird das Treibgas eingefüllt und die Aerosolverpackung druckdicht verschlossen.

Die Anwendung kann sowohl als Rinse-Produkt, als auch als Leave-in-Produkt erfolgen. Das erfindungsgemäße kosmetische Mittel wird angewendet, indem es in einer in Abhängigkeit von der Haarmenge und des Haarzustands zur Erzielung des gewünschten Haarpflegeeffektes ausreichenden Menge (typischerweise ca. 3 - 10 g) auf das gewaschene Haar als Schaum aufgebracht und verteilt wird. Nach einer Einwirkzeit (z.B. 3 - 6 Minuten) kann ausgespült werden. Ebenso kann das Mittel auch im Haar verbleiben und es wird nach der Anwendung getrocknet.

Die nachfolgenden Beispiele sollen den Gegenstand der Erfindung näher erläutern.

### Beispiele

### Beispiel 1

| | |
|---|---|
| Polyvinylpyrrolidon | 0,2 g |
| Polyquaternium-11 | 0,18 g |
| Cetyltrimethylammoniumchlorid | 0,25 g |
| Quaternium-80 | 0,45 g |
| MgSO₄ * 7 H₂O | 0,2 g |
| Isododekan | 2,5 g |
| Isobutan | 1,4 g |
| Butan | 6,8 g |
| Propan | 1,8 g |
| Wasser | Ad 100 g |

Die Zusammensetzung wird in einen druckfesten Behälter aus transparentem Polyethylenterephtalat abgefüllt. Der Behälter wird mit einem Schaumkopf versehen und verschlossen.

### Beispiel 2

| | |
|---|---|
| PVP/VA Copolymer | 0,35 g |
| Polyquaternium-11 | 0,24 g |
| Cetyltrimethylammoniumchlorid | 0,12 g |
| Quaternium-80 | 1,10 g |
| Natriumsulfat * 7 H₂O | 0,12 g |
| Cyclomethicone | 3,5 g |
| Isobutan | 1,2 g |
| Butan | 5,3 g |
| Propan | 1,5 g |
| Wasser | Ad 100 g |

Die Zusammensetzung wird in einen druckfesten Behälter aus transparentem Polyethylenterephtalat abgefüllt. Der Behälter wird mit einem Schaumkopf versehen und verschlossen.

### Beispiel 3

| | |
|---|---|
| Polyvinylpyrrolidon | 2,5 g |
| Polyquaternium-7 | 2,0 g |
| Cetyltrimethylammoniumchlorid | 0,55 g |
| Quaternium-80 | 1,60 g |
| Natriumchlorid | 0,45 g |
| Calciumacetat | 0,1 g |
| Ethanol | 6,0 g |
| Mineralöl | 2,8 g |
| Isobutan | 2,1 g |
| Butan | 10,2 g |
| Propan | 2,7 g |
| Wasser | Ad 100 g |

Die Zusammensetzung wird in einen druckfesten Behälter aus transparentem Polyethylenterephtalat abgefüllt. Der Behälter wird mit einem Schaumkopf versehen und verschlossen.

### Beispiel 4

| | |
|---|---|
| Polyvinylpyrrolidon | 0,45 g |
| Polyquaternium-16 | 0,75 g |
| Chitosan | 0,25 g |
| Milchsäure (90%ig) | 0,20 g |
| Cetyltrimethylammoniumchlorid | 0,55 g |
| Quaternium-80 | 1,60 g |
| Calciumacetat | 1,0 g |
| Jojobaöl | 1,5 g |
| Octyldodecanol | 2,0 g |
| Isobutan | 1,4 g |
| Butan | 6,8 g |
| Propan | 1,8 g |
| Wasser | Ad 100 g |

Die Zusammensetzung wird in einen druckfesten Behälter aus transparentem Polyethylenterephtalat abgefüllt. Der Behälter wird mit einem Schaumkopf versehen und verschlossen.

### Beispiel 5

| | |
|---|---|
| PVP/VA Copolymer | 3,8 g |
| Polyquaternium-11 | 0,18 g |
| Polyquaternium-16 | 0,82 g |
| Cetyltrimethylammoniumchlorid | 0,65 g |
| Quaternium-80 | 2,50 g |
| Calciumpantothenat | 0,65 g |
| Cetyl-/Stearylisononanoat | 1,0 g |
| Dicaprylylether | 2,50 g |
| Isostearinsäure | 1,50 g |
| Dimethylether | 1,0 g |
| Butan | 5,0 g |
| Propan | 4,0 g |
| Wasser | Ad 100 g |

Die Zusammensetzung wird in einen druckfesten Behälter aus transparentem Polyethylenterephtalat abgefüllt. Der Behälter wird mit einem Schaumkopf versehen und verschlossen.

### Beispiel 6: Vergleichsversuche

Zu der folgenden Grundrezeptur wurden verschiedene hydrophobe Substanzen und verschiedene Salze einzeln und in Kombination zugegeben. Es wurde untersucht, ob sich eine scharfe Phasentrennung zwischen zwei klaren, ungetrübten Phasen ausbildet. Dies war nur der Fall, wenn eine Kombination von hydrophober Substanz und Salz eingesetzt wurde. Die Ergebnisse sind in Tabelle 1 zusammengestellt. Mengenangaben beziehen sich auf das Gesamtgewicht der Zusammensetzung.

| | |
|---|---|
| Grundrezeptur: | |
| Polyvinylpyrrolidon | 0,2 g |
| (Luviskol^{®} K80 Pulver) | |
| Polyquaternium-11 | 1,0 g |
| Cetyltrimethylammoniumchlorid | 0,25 g |
| Quaternium-80 | 1,0 g |
| Glyoxylsäure | 0,05 g |
| Ethanol | 5,0 g |
| Wasser | Ad 100 g |
| Propan/Butan 90/10 ad 2,7 bar | |

**Tabelle 1:**

| Hydrophobe Substanz | Salz | Scharfe Phasentrennung |
|---|---|---|
| 3% Isododekan | - | Nein |
| - | 0,5% Na₂SO₄ | Nein |
| | 0,1% Na₂SO₄ | Nein |
| 3% Isododekan | 0,5% Na₂SO₄ | Ja |
| 3% Isododekan | 0,1% Na₂SO₄ | Ja |
| 3% Isododekan | 0,5% MgSO₄ | Ja |
| 3% Isododekan | 0,5% Ca-Acetat | Ja |
| 3% Isododekan | 0,5% NH₄Cl | Ja |
| 3% Isododekan | 0,5% K-Al-sulfat | Ja |
| 3% Isododekan | 0,5% Na₂SO₄ | Ja |
| 3% Isododekan | 0,5% Na₂Cl | Ja |
| 3% Isododekan | 0,5% CaCl₂ | Ja |
| 3% Isododekan | 0,5% Ca-Pantothenat | Ja |
| 3% Isododekan | 1% Na-Lactat | Ja |
| 3% Paraffinum perl. | - | Nein |
| 3% Paraffinum perl. | 0,25% Na₂SO₄ | Ja |
| Jojobaöl | - | Nein |
| Jojobaöl | 0,25% Na₂SO₄ | Ja |
| Isopropylmyristat | - | Nein |
| Isopropylmyristat | 0,25% Na₂SO₄ | Ja |
| Cetyl-/Stearyl-isononanoat | - | Nein |
| Cetyl-/Stearyl-isononanoat | 0,25% Na₂SO₄ | Ja |
| Cetyllactat | - | Nein |
| Cetyllactat | 0,25% Na₂SO₄ | Ja |
| Dicaprylylether | - | Nein |
| Dicaprylylether | 0,25% Na₂SO₄ | Ja |
| Diethylhexyl-cyclohexan | - | Nein |
| Diethylhexyl-cyclohexan | 0,25% Na₂SO₄ | Ja |
| Octyldodecanol | - | Nein |
| Octyldodecanol | 0,25% Na₂SO₄ | Ja |
| Cetyl Dimethicone | - | Nein |
| Cetyl Dimethicone | 0,25% Na₂SO₄ | Ja |
| Isostearinsäure | - | Nein |
| Isostearinsäure | 0,25% Na₂SO₄ | Ja |
| Caprylsäure | - | Nein |
| Caprylsäure | 0,25% Na₂SO₄ | Ja |
| Caprinsäure | - | Nein |
| Caprinsäure | 0,25% Na₂SO₄ | Ja |

## Patentansprüche

1. Produkt zur Haarbehandlung bestehend aus
(A) einer transparenten, druckfesten Aerosolverpackung,
(B) einer Vorrichtung zum Verschäumen einer in der Aerosolverpackung befindlichen Zusammensetzung,
(C) einer aus zwei flüssigen Phasen (D) und (E) bestehenden Wirkstoffmischung, welche bei Abgabe aus der Verpackung (A) mittels der Vorrichtung (B) einen Schaum bildet, wobei
(c1) beide flüssigen Phasen klar vorliegen,
(c2) beide flüssigen Phasen durch eine scharfe Phasengrenze voneinander getrennt vorliegen,
(D) eine der beiden flüssigen Phasen hydrophil ist und Wasser oder ein aus Wasser und wasserlöslichen organischen Lösungsmitteln gebildetes Lösungsmittelsystem enthält,
(d1) die hydrophile Phase mindestens einen kationischen oder kationaktiven haarpflegenden Stoff enthält,
(d2) die hydrophile Phase mindestens ein anorganisches Salz oder ein nicht-polymeres organisches Salz enthält, wobei das Kation des Salzes ein Metallion oder das Ammoniumion ist,
(E) die zweite der beiden flüssigen Phasen hydrophob ist und
(e1) mindestens ein wasserunlösliches und unter den in der Aerosolverpackung (A) herrschenden Druckverhältnissen in verflüssigter Form vorliegendes Treibgas enthält und
(e2) die hydrophobe Phase mindestens eine wasserunlösliche, in dem verflüssigten Treibgas (e1) lösliche hydrophobe Substanz enthält.

2. Produkt nach Anspruch 1, **dadurch gekennzeichnet, dass** das Material der Aerosolverpackung (A) ausgewählt ist aus Glas und transparenten, druckfesten Kunststoffen.

3. Produkt nach Anspruch 2, **dadurch gekennzeichnet, dass** das Material der Aerosolverpackung (A) Polyethylenterephtalat ist.

4. Produkt nach einem der vorhergehenden Ansprüche, **dadurch gekennzeichnet, dass** das Lösungsmittel der hydrophilen Phase (D) Wasser oder ein Gemisch aus Wasser, einwertigen C1- bis C4-Alkoholen und/oder mehrwertigen C2- bis C6-Alkoholen ist.

5. Produkt nach einem der vorhergehenden Ansprüche, **dadurch gekennzeichnet, dass** der kationische oder kationaktive haarpflegende Stoff (d1) ausgewählt ist aus kationischen Polymeren, kationischen Tensiden, kationischen Silikonverbindungen, kationisch derivatisierten Proteinen oder Proteinhydrolysaten und Betain mit jeweils mindestens einer kationischen oder kationaktiven Gruppe.

6. Produkt nach Anspruch 5, **dadurch gekennzeichnet, dass** das kationische Tensid ausgewählt ist aus Verbindungen der allgemeinen Formel (I)
N⁽⁺⁾R¹R²R³R⁴ X⁽⁻⁾ (I)
wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten, wobei mindestens einer der Reste R1 bis R4 mindestens 8 C-Atome aufweist und wobei X⁻ ein Anion darstellt.

7. Produkt nach Anspruch 5, **dadurch gekennzeichnet, dass** das kationische Polymer ausgewählt ist aus Methylvinylimidazoliumchlorid/Vinylpyrrolidon Copolymeren, quaternisierten Vinylpyrrolidon/Dimethylaminoethylmethacrylat Copolymeren, kationisch derivatisierten Polysacchariden, Chitosan, Chitosansalzen und Chitosanderivaten.

8. Produkt nach Anspruch 5, **dadurch gekennzeichnet, dass** die kationische Silikonverbindung ausgewählt ist aus endständig diquaternären Polydimethylsiloxanen.

9. Produkt nach einem der vorhergehenden Ansprüche, **dadurch gekennzeichnet, dass** der kationische oder kationaktive haarpflegende Stoff (d1) in einer Menge von 0,05 bis 10 Gew.% bezogen auf die Gesamtzusammensetzung enthalten ist.

10. Produkt nach einem der vorhergehenden Ansprüche, **dadurch gekennzeichnet, dass** das organische oder anorganische Salz (d2) ausgewählt ist aus Salzen, bei denen das Kation aus ein-, zwei- oder dreiwertigen Metallionen oder aus dem Ammoniumion gebildet wird und bei denen das Anion ausgewählt ist aus Chlorid, Sulfat, Phosphat oder einer deprotonierten organischen Säure.

11. Produkt nach einem der vorhergehenden Ansprüche, **dadurch gekennzeichnet, dass** das organische oder anorganische Salz (d2) in einer Menge von 0,05 bis 2 Gew.% bezogen auf die Gesamtzusammensetzung enthalten ist.

12. Produkt nach einem der vorhergehenden Ansprüche, **dadurch gekennzeichnet, dass** das Treibgas (e1) ausgewählt ist aus C3- bis C5-Kohlenwasserstoffen, deren Gemischen sowie aus Mischungen von einem oder mehreren C3- bis C5-Kohlenwasserstoffen mit Dimethylether.

13. Produkt nach einem der vorhergehenden Ansprüche, **dadurch gekennzeichnet, dass** die Menge an Treibgas (e1) so gewählt ist, dass der Druck in der Aerosolverpackung (A) maximal 2,7 bar bei 20 °C beträgt.

14. Produkt nach einem der vorhergehenden Ansprüche, **dadurch gekennzeichnet, dass** die hydrophobe Substanz (e2) öl- oder fettartig ist und ausgewählt ist aus pflanzlichen, tierischen, synthetischen, oder mineralischen Ölen, flüssigen Kohlenwasserstoffen, Fettalkoholen, Fettalkoholestern, Fettsäuren, Fettsäureestern, Fettalkoholethern und flüssigen Silikonverbindungen.

15. Produkt nach einem der vorhergehenden Ansprüche, **dadurch gekennzeichnet, dass** die hydrophobe Substanz (e2) ausgewählt ist aus flüchtigen Kohlenwasserstoffen und flüchtigen Silikonverbindungen.

16. Produkt nach einem der vorhergehenden Ansprüche, **dadurch gekennzeichnet, dass** die hydrophobe Substanz (e2) in einer Menge von 0,5 bis 10 Gew.% bezogen auf die Gesamtzusammensetzung enthalten ist.

17. Produkt nach einem der vorhergehenden Ansprüche, **dadurch gekennzeichnet, dass** es zusätzlich mindestens ein nicht-ionisches filmbildendes Polymer enthält.

18. Produkt nach Anspruch 17, **dadurch gekennzeichnet, dass** das nichtionische Polymer ausgewählt ist aus Polyvinylpyrrolidon und Polyvinylpyrrolidon/Vinylacetat Copolymeren.

19. Produkt nach Anspruch 17 oder 18, **dadurch gekennzeichnet, dass** das nichtionische Polymer in einer Menge von 0,05 bis 5 Gew.% bezogen auf die Gesamtzusammensetzung enthalten ist.

20. Produkt nach einem der vorhergehenden Ansprüche, **dadurch gekennzeichnet, dass** in der hydrophoben Phase mindestens ein erster, wasserunlöslicher, öllöslicher Anfärbefarbstoffe enthalten ist und in der hydrophilen Phase mindestens ein zweiter, zu dem ersten Anfärbefarbstoff verschiedenfarbiger, ölunlöslicher, wasserlöslicher bzw. in Wasser/Alkohol-Gemischen löslicher Anfärbefarbstoffe enthalten ist.

21. Produkt nach einem der vorhergehenden Ansprüche, **dadurch gekennzeichnet, dass** in der Wirkstoffzusammensetzung
(h) 0,05 bis 5,0 Gew.% mindestens eines wasserlöslichen, nichtionischen, filmbildenden Polymers
(i) 0,05 bis 5,0 Gew.% mindestens eines wasserlöslichen kationischen Polymers und/oder mindestens eines diquaternären Polydimethylsiloxans,
(j) 0,05 bis 2,5 Gew.% mindestens eines wasserlöslichen kationischen Tensids
(k) 0,05 bis 2,0 Gew.% mindestens eines wasserlöslichen anorganischen Salzes und/oder eines nicht-polymeren organischen Salzes, wobei das Kation des Salzes ein Metallion oder das Ammoniumion ist
(l) 63,0 bis 97,25 Gew. Wasser oder eines Wasser/ Alkohol-Gemisches,
(m) 0,5 bis 5,0 Gew.% mindestens eines unter Normalbedingungen flüssigen, flüchtigen Kohlenwasserstoffs und/oder eines flüchtigen Silikons und
(n) 2,0 bis 20,0 Gew.% eines Treibmittels, ausgewählt aus Propan, Butanen, deren Gemischen und deren Gemischen mit Dimethylether,
wobei sich die Mengenangaben auf die Gesamtzusammensetzung beziehen.

## Claims

1. Product for hair treatment, composed of
(A) a transparent, pressure-resistant aerosol container,
(B) a device for foaming a composition contained in the aerosol container,
(C) an effective ingredient mixture which is composed of two liquid phases (D) and (E) and which when dispensed from the container (A) by means of the device (B) forms a foam, wherein
(c1) both liquid phases are clear,
(c2) both liquid phases are separated from one another by a sharp phase boundary,
(D) one of the two liquid phases is hydrophilic and comprises water or a solvent system formed from water and water-soluble organic solvents,
(d1) the hydrophilic phase comprises at least one cationic or cationically-active hair care ingredient,
(d2) the hydrophilic phase comprises at least one inorganic salt or one non-polymeric organic salt, the cation of the salt being a metal ion or the ammonium ion,
(E) the second of the two liquid phases is hydrophobic and
(e1) comprises at least one water-insoluble propellant gas present in liquefied form under the pressure conditions present in the aerosol container (A), and
(e2) the hydrophobic phase comprises at least one water-insoluble hydrophobic substance soluble in the liquefied propellant gas (e1).

2. Product according to Claim 1, **characterized in that** the material of the aerosol container (A) is selected from glass and transparent, pressure-resistant plastics.

3. Product according to Claim 2, **characterized in that** the material of the aerosol container (A) is polyethylene terephthalate.

4. Product according to any one of the preceding claims, **characterized in that** the solvent of the hydrophilic phase (D) is water or a mixture of water, monohydric C1 to C4 alcohols and/or polyhydric C2 to C6 alcohols.

5. Product according to any one of the preceding claims, **characterized in that** the cationic or cationically-active hair care ingredient (d1) is selected from cationic polymers, cationic surfactants, cationic silicone compounds, cationically derivatized proteins or protein hydrolyzates and betaine having in each case at least one cationic or cationically-active group.

6. Product according to Claim 5, **characterized in that** the cationic surfactant is selected from compounds of the general formula (I)
N⁽⁺⁾R¹R²R³R⁴ X⁽⁻⁾ (I)
wherein R1 to R4 independently of each other are aliphatic groups, aromatic groups, alkoxy groups, polyoxyalkylene groups, alkylamido groups, hydroxyalkyl groups, aryl groups or alkaryl groups with 1 to 22 carbon atoms, at least one of the radicals R1 to R4 having at least 8 carbon atoms, and X⁻ representing an anion.

7. Product according to Claim 5, **characterized in that** the cationic polymer is selected from methylvinylimidazolium chloride/vinylpyrrolidone copolymers, quaternized vinylpyrrolidone/dimethylaminoethyl methacrylate copolymers, cationically derivatized polysaccharides, chitosan, chitosan salts and chitosan derivatives.

8. Product according to Claim 5, **characterized in that** the cationic silicone compound is selected from terminally diquaternary polydimethylsiloxanes.

9. Product according to any one of the preceding claims, **characterized in that** the cationic or cationically-active hair care ingredient (d1) is contained in an amount from 0.05% to 10% by weight, based on the total composition.

10. Product according to any one of the preceding claims, **characterized in that** the organic or inorganic salt (d2) is selected from salts wherein the cation is formed of monovalent, divalent or trivalent metal ions or of the ammonium ion and wherein the anion is selected from chloride, sulfate, phosphate or a deprotonated organic acid.

11. Product according to any one of the preceding claims, **characterized in that** the organic or inorganic salt (d2) is contained in an amount from 0.05% to 2% by weight, based on the total composition.

12. Product according to any one of the preceding claims, **characterized in that** the propellant gas (e1) is selected from C3 to C5 hydrocarbons, mixtures thereof, and mixtures of one or more C3 to C5 hydrocarbons with dimethyl ether.

13. Product according to any one of the preceding claims, **characterized in that** the amount of propellant gas (e1) is selected such that the pressure in the aerosol container (A) is at most 2.7 bar at 20°C.

14. Product according to any one of the preceding claims, **characterized in that** the hydrophobic substance (e2) is oil-like or fatlike and is selected from plant oils, animal oils, synthetic oils or mineral oils, liquid hydrocarbons, fatty alcohols, fatty alcohol esters, fatty acids, fatty acid esters, fatty alcohol ethers and liquid silicone compounds.

15. Product according to any one of the preceding claims, **characterized in that** the hydrophobic substance (e2) is selected from volatile hydrocarbons and volatile silicone compounds.

16. Product according to any one of the preceding claims, **characterized in that** the hydrophobic substance (e2) is contained in an amount from 0.5% to 10% by weight, based on the total composition.

17. Product according to any one of the preceding claims, **characterized in that** it further comprises at least one non-ionic film-forming polymer.

18. Product according to Claim 17, **characterized in that** the non-ionic polymer is selected from polyvinylpyrrolidone and polyvinylpyrrolidone/vinyl acetate copolymers.

19. Product according to Claim 17 or 18, **characterized in that** the non-ionic polymer is contained in an amount from 0.05% to 5% by weight, based on the total composition.

20. Product according to any one of the preceding claims, **characterized in that** in the hydrophobic phase there is at least one first, water-insoluble, oil-soluble tinting dye and in the hydrophilic phase there is at least one tinting dye which is different in colour from the first tinting dye and is insoluble in oil, soluble in water and/or soluble in water/alcohol mixtures.

21. Product according to any one of the preceding claims, **characterized in that** the effective ingredient composition comprises
(h) 0.05% to 5.0% by weight of at least one water-soluble non-ionic film-forming polymer,
(i) 0.05% to 5.0% by weight of at least one water-soluble cationic polymer and/or at least one diquaternary polydimethylsiloxane,
(j) 0.05% to 2.5% by weight of at least one water-soluble cationic surfactant,
(k) 0.05% to 2.0% by weight of at least one water-soluble inorganic salt and/or one non-polymeric organic salt, the cation of the salt being a metal ion or the ammonium ion,
(l) 63.0% to 97.25% by weight of water or a water/alcohol mixture,
(m) 0.5% to 5.0% by weight of at least one volatile hydrocarbon which is liquid under standard conditions and/or one volatile silicone, and
(n) 2.0% to 20.0% by weight of a propellant, selected from propane, butanes, mixtures thereof and mixtures thereof with dimethyl ether,
the amounts being based on the total composition.

## Revendications

1. Produit pour le traitement capillaire, constitué
(A) d'un emballage aérosol transparent, résistant à la pression,
(B) d'un dispositif destiné à faire mousser une composition se trouvant dans l'emballage aérosol,
(C) d'un mélange de substances actives consistant en deux phases liquides (D) et (E), qui forme une mousse lors de la libération hors de l'emballage (A) au moyen du dispositif (B),
(c1) les deux phases liquides étant limpides,
(c2) les deux phases liquides étant séparées l'une de l'autre par une nette limite de phases,
(D) l'une des deux phases liquides est hydrophile et contient de l'eau ou un système de solvants constitué d'eau et de solvants organiques hydrosolubles,
(d1) la phase hydrophile contient au moins une substance de soin capillaire, cationique ou activité cationique,
(d2) la phase hydrophile contient au moins un sel minéral ou un sel organique non polymère, le cation du sel étant un ion métallique ou l'ion ammonium,
(E) la seconde des deux phases liquides est hydrophobe et
(e1) contient au moins un gaz propulseur insoluble dans l'eau et se trouvant sous forme liquéfiée dans les conditions de pression régnant dans l'emballage aérosol (A) et
(e2) la phase hydrophobe contient au moins une substance hydrophobe insoluble dans l'eau, soluble dans le gaz propulseur (e1) liquéfié.

2. Produit selon la revendication 1, **caractérisé en ce que** le matériau de l'emballage aérosol (A) est choisi parmi le verre et des matières plastiques transparentes, résistant à la pression.

3. Produit selon la revendication 2, **caractérisé en ce que** le matériau de l'emballage aérosol (A) est le poly(éthylène-téréphtalate).

4. Produit selon l'une quelconque des revendications précédentes, **caractérisé en ce que** le solvant de la phase hydrophile (D) est l'eau ou un mélange d'eau, d'alcools en C₁-C₄ monovalents et/ou d'alcools en C₂-C₆ plurivalents.

5. Produit selon l'une quelconque des revendications précédentes, **caractérisé en ce que** la substance cationique ou à activité cationique (d1) est choisie parmi des polymères cationiques, des tensioactifs cationiques, des composés silicone cationiques, des protéines ou des hydrolysats de protéines à fonctionnalisation cationique et la bétaïne, comportant chacun au moins un groupe cationique ou à activité cationique.

6. Produit selon la revendication 5, **caractérisé en ce que** le tensioactif cationique est choisi parmi des composés de formule générale (I)
N⁽⁺⁾R¹R²R³R⁴ X⁽⁻⁾ (I)
dans laquelle R¹ à R⁴ représentent, indépendamment les uns des autres, des groupes aliphatiques, des groupes aromatiques, des groupes alcoxy, des groupes polyoxyalkylène, des groupes alkylamido, des groupes hydroxyalkyle, des groupes aryle ou alkaryle ayant de 1 à 22 atomes de carbone, au moins l'un des radicaux R¹ à R⁴ comportant au moins 8 atomes de carbone et X⁽⁻⁾ représente un anion.

7. Produit selon la revendication 5, **caractérisé en ce que** le polymère cationique est choisi parmi des copolymères chlorure de méthylvinylimidazolium/vinylpyrrolidone, des copolymères vinylpyrrolidone/méthacrylate de diméthylaminoéthyle rendus quaternaires, des polysaccharides à fonctionnalisation cationique, le chitosane, des sels de chitosane et des dérivés de chitosane.

8. Produit selon la revendication 5, **caractérisé en ce que** le composé silicone cationique est choisi parmi des polydiméthylsiloxanes diquaternaires en bout de chaîne.

9. Produit selon l'une quelconque des revendications précédentes, **caractérisé en ce que** la substance de soin capillaire (d1) cationique ou à activité cationique est contenue en une quantité de 0,05 à 10 % en poids, par rapport à la composition totale.

10. Produit selon l'une quelconque des revendications précédentes, **caractérisé en ce que** le sel organique ou minéral (d2) est choisi parmi les sels dans lesquels le cation consiste en des ions métalliques mono-, di- ou trivalents ou en l'ion ammonium et dans lesquels l'anion est choisi parmi le chlorure, le sulfate, le phosphate et un acide organique déprotoné.

11. Produit selon l'une quelconque des revendications précédentes, **caractérisé en ce que** le sel organique ou minéral (d2) est contenu en une quantité de 0,05 à 2 % en poids par rapport à la composition totale.

12. Produit selon l'une quelconque des revendications précédentes, **caractérisé en ce que** le gaz propulseur (e1) est choisi parmi des hydrocarbures en C₃-C₅, leurs mélanges ainsi que des mélanges d'un ou plusieurs hydrocarbures en C₃-C₅ avec l'éther diméthylique.

13. Produit selon l'une quelconque des revendications précédentes, **caractérisé en ce que** la quantité du gaz propulseur (e1) est choisie de manière que la pression dans l'emballage aérosol (A) soit d'au maximum 2,7 bars à 20 °C.

14. Produit selon l'une quelconque des revendications précédentes, **caractérisé en ce que** la substance hydrophobe (e2) est de type huile ou graisse et est choisie parmi des huiles végétales, animales, synthétiques ou minérales, des hydrocarbures liquides, des alcools gras, des esters d'alcools gras, des acides gras, des esters d'acides gras, des éthers d'alcools gras et des composés silicone liquides.

15. Produit selon l'une quelconque des revendications précédentes, **caractérisé en ce que** la substance hydrophobe (e2) est choisie parmi des hydrocarbures liquides et des composés silicone liquides.

16. Produit selon l'une quelconque des revendications précédentes, **caractérisé en ce que** la substance hydrophobe (e2) est contenue en une quantité de 0,5 à 10 % en poids, par rapport à la composition totale.

17. Produit selon l'une quelconque des revendications précédentes, **caractérisé en ce qu'**en outre il contient au moins un polymère filmogène non ionique.

18. Produit selon la revendication 17, **caractérisé en ce que** le polymère non ionique est choisi parmi la polyvinylpyrrolidone et des copolymères polyvinylpyrrolidone/acétate de vinyle.

19. Produit selon la revendication 17 ou 18, **caractérisé en ce que** le polymère non ionique est contenu en une quantité de 0,05 à 5 % en poids, par rapport à la composition totale.

20. Produit selon l'une quelconque des revendications précédentes, **caractérisé en ce qu'**au moins un premier colorant pour coloration, liposoluble, insoluble dans l'eau, est contenu dans la phase hydrophobe et au moins un second colorant pour coloration, insoluble dans l'huile, hydrosoluble ou soluble dans des mélanges eau/alcool, de couleur différente de celle du premier colorant, est contenu dans la phase hydrophile.

21. Produit selon l'une quelconque des revendications précédentes, **caractérisé en ce que** la composition de substances actives contient
(h) 0,05 à 5,0 % en poids d'un polymère filmogène non ionique hydrosoluble,
(i) 0,05 à 5,0 % en poids d'au moins un polymère cationique hydrosoluble et/ou d'au moins un polydiméthylsiloxane diquaternaire,
(j) 0,05 à 2,5 % en poids d'au moins un tensioactif cationique hydrosoluble,
(k) 0,05 à 2,0 % en poids d'au moins un sel minéral hydrosoluble et/ou d'un sel organique non polymère, le cation du sel étant un ion métallique ou l'ion ammonium,
(l) 63,0 à 97,25 % en poids d'eau ou d'un mélange eau/alcool,
(m) 0,5 à 5,0 % en poids d'au moins un hydrocarbure volatil, liquide dans les conditions normales et/ou d'un silicone volatil et
(n) 2,0 à 20,0 % en poids d'un propulseur choisi parmi le propane, les butane, des mélanges de ceux-ci et des mélanges de ceux-ci avec l'éther diméthylique,
les données quantitatives se rapportant à la composition totale.
